# EUROPEAN PATENT SPECIFICATION

(11) **EP 3 660 005 B1**
(45) Date of publication and mention of the grant of the patent: **29.06.2022**
(21) Application number: 18215021.9
(22) Date of filing: 21.12.2018
(51) Int. Cl.: C07D 239/52

(54) **METHOD FOR PREPARING AZOXYSTROBIN**
VERFAHREN ZUR HERSTELLUNG VON AZOXYSTROBIN
PROCÉDÉ DE PRÉPARATION D'AZOXYSTROBINE

(30) Priority: 28.11.2018 TW 107142511
(43) Date of publication of application: 03.06.2020
(73) Proprietor: Sinon Corporation, Taichung City 43245 (TW)
(72) Inventor: CHEN, Chien-Hsing, 43245 TAICHUNG CITY (TW); HSIEH, Ming-Fang, 43245 TAICHUNG CITY (TW); LIN, Chih-Da, 43245 TAICHUNG CITY (TW); LIU, Chien-Yu, 43245 TAICHUNG CITY (TW)
(74) Representative: Straus, Alexander

(56) References cited:
- WO-A1-2008/043977
- WO-A1-2014/190997

## Description

### BACKGROUND OF THE INVENTION

### 1. Field of the Invention

The present invention relates to a method for preparing a fungicidal agent, and particularly to a method for preparing azoxystrobin.

### 2. Description of the Related Art

The compound methyl (E)-2-[2-(6-(2-cyanophenoxy)pyrimidin-4-yloxy)phenyl]-3-methoxyacrylate is a widely used fungicidal agent in agriculture known as Azoxystrobin.

PCT Publication No. WO 9208703 discloses that the derivative of phenoxypyrimidine has fungicidal potency.

Moreover, US Patent No. US5395837 discloses a method for synthesizing a 4,6-bis(aryloxy)pyrimidine derivative, by reacting at 95 - 100°C in N,N-dimethylformamide (DMF) as a solvent in the presence of potassium carbonate as a base and cuprous chloride (CuCl) as a catalyst. The yield is 64%, and the reaction scheme is as follows:

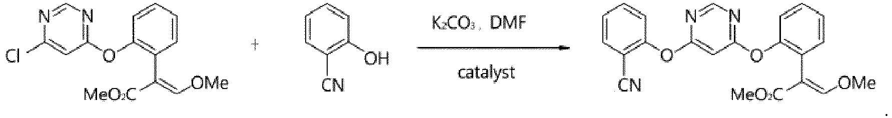

Furthermore, Bayer further discloses in PCT Publication No. WO 0172719 that a phenoxy derivative and a chloropyrimidine derivative are reacted at 50-80°C in methyl isobutyl ketone as a solvent, in the presence of potassium carbonate as a base and 1,4-diazabicyclo[2,2,2]octane (DABCO) as a catalyst. The yield is 79 - 96%.

Then, Syngenta also discloses in EP Publication No. EP 1891020 that in the preparation of azoxystrobin, the yield is 86-98% when 0.1-40 mol% DABCO is added as a catalyst. The reaction scheme is as follows:

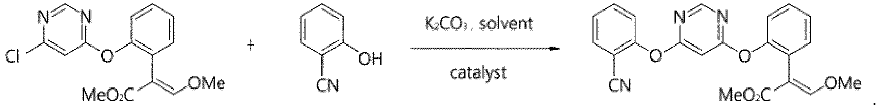

WO 2008043977 A1 discloses a process for preparing asymmetrical 4,6-bis(aryloxy)pyrimidines derivatives, in particular for preparing strobilurin fungicides such as methyl (E)-2-{2-[6-(2-cyanophenoxy)pyrimidin-4-yloxy]phenyl}-3-methoxy-acrylate (azoxystrobin) using a quinuclidine-based catalyst or an optionally 3-substituted N-methyl pryrrolidine-based catalyst.

WO 2014190997 discloses a process for producing 4,6- bis(aryloxy)pyrimidines derivatives in presence of N-methylpyrrolidine, while adding water and toluene after reaction, to perform a phase separation.

Therefore, as can be known from the above-mentioned prior art that although DABCO is previously often used as a catalyst, DABCO cannot be easily recycled and reused, and thus is not a suitable catalyst.

### SUMMARY OF THE INVENTION

A main object of the present invention is to provide a method for preparing azoxystrobin by using a catalyst that is easy to recycle and reuse in a process and is thus environmentally friendly. The present method is an environmentally friendly preparation method.

To achieve the above object, the present invention provides a method for preparing azoxystrobin, which comprises Step (a): mixing methyl (E)-2-[2-(6-chloropyrimidin-4-yloxy)phenyl]-3-methoxyacrylate, 2-cyanophenol, potassium carbonate, and 10-80 mol% of 1-methylpyrrolidine as a catalyst in an aprotic solvent to form a basic mixture, and reacting the basic mixture for 2-5 hrs at a temperature of 60-120°C; and Step (b): subjecting the basic mixture after reaction in Step (a) to a first distillation under a reduced pressure of 10666-15999 Pa (80-120 Torr) at 70-80°C, to obtain azoxystrobin.

Therefore, in the method for preparing azoxystrobin provided in the present invention, 1-methylpyrrolidine is used as a catalyst that is easy to recycle and reuse in a process and is thus environmentally friendly. The present method is an environmentally friendly preparation method.

### DETAILED DESCRIPTION OF THE PREFERRED EMBODIMENTS

Step (a) comprises mixing methyl (E)-2-[2-(6-chloropyrimidin-4-yloxy)phenyl]-3-methoxyacrylate, 2-cyanophenol, potassium carbonate, and 10-80 mol% of 1-methylpyrrolidine as a catalyst in an aprotic solvent, to form a basic mixture; and reacting the basic mixture for 2-5 hrs at a temperature of 60-120°C, thus providing a preferred reaction environment for the basic mixture.

In Step (a), considering the effect and cost, the amount of the catalyst 1-methylpyrrolidine is preferably 10-80 mol%; preferably, the aprotic solvent is toluene, N,N-dimethylformamide, or methyl isobutyl ketone; and the basic mixture is azeotropically predistilled at 50-60°C under 13332-15999 Pa (100-120 Torr) to remove water, whereby the yield of azoxystrobin is improved effectively. However, the removal of water by azeotropic predistillation is not necessary.

Step (b) comprises subjecting the basic mixture after reaction in Step (a) to a first distillation under a reduced pressure of 10666-15999 Pa (80-120 Torr) at 70-80°C, to obtain azoxystrobin.

Step (c) comprises adding the product left after the first distillation under reduced pressure to the aprotic solvent and water, mixing, and standing for layer separation, collecting an upper organic layer, subjecting the organic layer to a second distillation under a reduced pressure of 2666-5333 Pa (20-40 Torr) at 50-60°C to obtain a crude product, dissolving the crude product in methanol at 30-65°C, cooling to normal temperature and then to 0-5°C, filtering to obtain a crystalline solid, washing the crystalline solid with methanol, and then drying the crystalline solid at 40-65°C.

Step (c) is intended to enhance the purity of azoxystrobin. Where no high purity of azoxystrobin is required, Step (c) can be omitted. Therefore, Step (c) is not necessary in the present invention.

To explain the technical features of the present invention in detail, the present invention is described below with reference to preferred embodiments.

In a first preferred embodiment of the present invention, a method for preparing azoxystrobin is provided, which comprises mainly the following steps.

Methyl (E)-2-[2-(6-chloropyrimidin-4-yloxy)phenyl]-3-methoxyacrylate (97.6 gm), 2-cyanophenol (36.9 gm), and potassium carbonate (25.4 gm) are added to, mixed, and dissolved in an aprotic solvent (241 gm) at room temperature. In this embodiment, the aprotic solvent is, for example, toluene. Then, 1-methylpyrrolidine (10.2 gm, 0.4 eq) is added as a catalyst, and stirred until uniform to form a basic mixture. Subsequently, the basic mixture is heated to 80°C, and reacted for 3 hrs.

The basic mixture after reaction is subjected to a first distillation under a reduced pressure of 13332 Pa (100 Torr) at 80°C, and the product left after the first distillation under reduced pressure is azoxystrobin. The distillate by the first distillation under reduced pressure is detected by gas chromatography (GC), and 1-methylpyrrolidine is determined to have a recovery rate of 96.3%.

Azoxystrobin left after the first distillation under reduced pressure is added to the aprotic solvent (241 gm) and water (114 gm), mixed, and stirred until azoxystrobin is completely dissolved. After standing for layer separation, an upper first organic layer is collected, and then a lower aqueous layer is collected and further added with the aprotic solvent (15 gm). Next, an upper second organic layer is collected. The first organic layer and the second organic layer are combined, and detected by high performance liquid chromatography (HPLC) to show a yield of azoxystrobin of 94.9%. The combined organic layer is subjected to a second distillation under reduced pressure at 50-60°C under 2666-5333 Pa (20-40 Torr), to remove the solvent. This gives a crude product. The crude product is dissolved in methanol (156 gm) at 60°C, cooled to normal temperature and then to 5°C, and filtered to obtain a crystalline solid. The crystalline solid was washed with methanol (13 gm) and then dried at 50°C, to obtain purified azoxystrobin (112.9 gm) as a solid (yield 91.5%).

The reaction scheme is as follows:

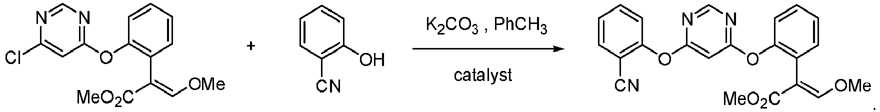

In a second preferred embodiment of the present invention, a method for preparing azoxystrobin is provided, which is substantially the same as that in the first preferred embodiment, except for the following differences.

The amount of the catalyst 1-methylpyrrolidine is (2.6 gm, 0.1 eq), the basic mixture is heated to 80°C, and the reaction time is 5 hrs.

The yield of azoxystrobin is 91.6%, and the recovery rate of the catalyst 1-methylpyrrolidine is 96.9%.

The yield of the resulting solid azoxystrobin (109.0 gm) is 88.3%.

In a third preferred embodiment of the present invention, a method for preparing azoxystrobin is provided, which is substantially the same as that in the first preferred embodiment, except for the following differences.

The amount of the catalyst 1-methylpyrrolidine is (5.1 gm, 0.2 eq), the basic mixture is heated to 80°C, and the reaction time is 5 hrs.

The yield of azoxystrobin is 91.9%, and the recovery rate of the catalyst 1-methylpyrrolidine is 97.1%.

The yield of the resulting solid azoxystrobin (109.4 gm) is 88.6%.

In a fourth preferred embodiment of the present invention, a method for preparing azoxystrobin is provided, which is substantially the same as that in the first preferred embodiment, except for the following differences.

The amount of the catalyst 1-methylpyrrolidine is (20.3 gm, 0.8 eq), the basic mixture is heated to 80°C, and the reaction time is 3 hrs.

The yield of azoxystrobin is 95.1%, and the recovery rate of the catalyst 1-methylpyrrolidine is 93.7%.

The yield of the resulting solid azoxystrobin (113.2 gm) is 91.7%.

In a fifth preferred embodiment of the present invention, a method for preparing azoxystrobin is provided, which is substantially the same as that in the first preferred embodiment, except for the following differences.

The basic mixture is heated to 120°C, and the reaction time is 2 hrs.

The yield of azoxystrobin is 93.1%, and the recovery rate of the catalyst 1-methylpyrrolidine is 87.2%.

The yield of the resulting solid azoxystrobin (110.8 gm) is 89.8%.

In a sixth preferred embodiment of the present invention, a method for preparing azoxystrobin is provided, which is substantially the same as that in the first preferred embodiment, except for the following differences.

The aprotic solvent is methyl isobutyl ketone (241 gm).

The yield of azoxystrobin is 95.4%, and the recovery rate of the catalyst 1-methylpyrrolidine is 95.9%.

The yield of the resulting solid azoxystrobin (113.6 gm) is 92.0%.

In a seventh preferred embodiment of the present invention, a method for preparing azoxystrobin is provided, which is substantially the same as that in the first preferred embodiment, except for the following differences.

The aprotic solvent is N,N-dimethylformamide (241 gm).

The yield of azoxystrobin is 92.8%, and the recovery rate of the catalyst 1-methylpyrrolidine is 96.8%.

The yield of the resulting solid azoxystrobin (110.5 gm) is 89.5%.

In an eighth preferred embodiment of the present invention, a method for preparing azoxystrobin is provided, which is substantially the same as that in the first preferred embodiment, except for the following differences.

The amount of the catalyst 1-methylpyrrolidine is (5.1 gm, 0.2 eq), triethylamine (6.1 gm, 0.2 eq) is added, the basic mixture is heated to 80°C, and the reaction time is 5 hrs.

The yield of azoxystrobin is 92.9%, and the recovery rate of the catalyst 1-methylpyrrolidine is 96.1%.

The yield of the resulting solid azoxystrobin (110.6 gm) is 89.6 %.

In a ninth preferred embodiment of the present invention, a method for preparing azoxystrobin is provided, which is substantially the same as that in the first preferred embodiment, except for the following differences.

Removal of water by azeotropic distillation (at 60°C under 13332 Pa (100 Torr)) is performed, the catalyst 1-methylpyrrolidine is added, the basic mixture is heated to 80°C, and the reaction time is 4 hrs.

The yield of azoxystrobin is 97.3%, and the recovery rate of the catalyst 1-methylpyrrolidine is 96.4%. Therefore, the basic mixture is reacted in the absence of water to effectively increase the yield of azoxystrobin.

The yield of the resulting solid azoxystrobin (115.8 gm) is 93.8%.

In a tenth preferred embodiment of the present invention, a method for preparing azoxystrobin is provided, which is substantially the same as that in the first preferred embodiment, except for the following differences.

The catalyst 1-methylpyrrolidine recovered from the process is used, the amount of the catalyst 1-methylpyrrolidine is (10.2 gm, 0.4 eq), the basic mixture is heated to 80°C, and the reaction time is 3 hrs.

The yield of azoxystrobin is 94.1%, and the recovery rate of the catalyst 1-methylpyrrolidine is 95.5%. This indicates that the catalyst 1-methylpyrrolidine recovered from the process can be repeatedly used.

The yield of the resulting solid azoxystrobin (111.9 gm) is 90.7 %.

It can be known from the results of the above preferred embodiments that in the method for preparing azoxystrobin provided in the present invention, 1-methylpyrrolidine is used as a catalyst that is easy to recycle and reuse in a process and is thus environmentally friendly. The present method is an environmentally friendly preparation method.

## Claims

1. A method for preparing azoxystrobin, the method being **characterized in that** the method comprising the steps (a) and (b):
(a) mixing methyl (E)-2-[2-(6-chloropyrimidin-4-yloxy)phenyl]-3-methoxyacrylate, 2-cyanophenol, potassium carbonate, and 10-80 mol% of 1-methylpyrrolidine as a catalyst in an aprotic solvent, to form a basic mixture; and reacting the basic mixture for 2-5 hrs at a temperature of 60-120°C; and
(b) subjecting the basic mixture in Step (a) to a first distillation under a reduced pressure of 10666-15999 Pa (80-120 Torr) at 70-80°C, to obtain azoxystrobin.

2. The preparation method according to claim 1, further comprising the step of:
(c) adding the product left after the first distillation under reduced pressure to the aprotic solvent and water, mixing, and standing for layer separation, collecting an upper organic layer, subjecting the organic layer to a second distillation under a reduced pressure of 2666-5333 Pa (20-40 Torr) at 50-60°C to obtain a crude product, dissolving the crude product in methanol at 30-65°C, cooling to normal temperature and then to 0-5°C, filtering to obtain a crystalline solid, washing the crystalline solid with methanol, and then drying the crystalline solid at 40-65°C.

3. The preparation method according to claim 1, wherein the aprotic solvent is toluene, N,N-dimethylformamide, or methyl isobutyl ketone.

4. The preparation method according to claim 1, wherein the basic mixture is reacted for 2-5 hrs at a temperature of 80-120°C.

5. The preparation method according to claim 1, wherein in Step (a), the basic mixture is reacted for 3 hrs at a temperature of 80°C; and in Step (b), the first distillation under reduced pressure occurs at 80°C under 13332 Pa (100 Torr).

6. The preparation method according to claim 1, wherein in Step (a), the basic mixture is reacted for 2 hrs at a temperature of 120°C; and in Step (b), the first distillation under reduced pressure occurs at 80°C under 13332 Pa (100 Torr).

7. The preparation method according to claim 1, wherein in step (a),
the basic mixture is azeotropically pre-distilled at 50-60°C under 13332-15999 Pa (100-120 Torr) to remove water; or
the mixed components of the basic mixture without the 1-methylpyrrolidine are azeotropically pre-distilled at 60°C under 13332 Pa (100 Torr) prior to adding 1-methylpyrrolidine to form the basic mixture, wherein the basic mixture is heated to 80°C for 4 hrs.

## Patentansprüche

1. Verfahren zur Herstellung von Azoxystrobin, wobei das Verfahren **dadurch gekennzeichnet ist, dass** das Verfahren die Schritte (a) und (b) umfasst:
(a) Mischen von Methyl-(E)-2-[2-(6-chlorpyrimidin-4-yloxy)phenyl]-3-methoxyacrylat, 2-Cyanophenol, Kaliumcarbonat und 10-80 mol-% 1-Methylpyrrolidin als Katalysator in einem aprotischen Lösungsmittel, um ein basisches Gemisch zu bilden; und Umsetzen des basischen Gemisches für 2-5 Stunden bei einer Temperatur von 60-120°C; und
(b) Durchführen einer ersten Destillation an der basischen Mischung aus Schritt (a) unter einem reduzierten Druck von 10666-15999 Pa (80-120 Torr) bei 70-80°C, um Azoxystrobin zu erhalten.

2. Herstellungsverfahren nach Anspruch 1, das zudem den Schritt umfasst:
(c) Zugeben des nach der ersten Destillation unter vermindertem Druck verbliebenen Produkts zu dem aprotischen Lösungsmittel und Wasser, Mischen und Stehenlassen zur Schichtentrennung, Gewinnen einer oberen organischen Schicht, Durchführen einer zweiten Destillation an der organischen Schicht unter einem verminderten Druck von 2666-5333 Pa (20-40 Torr) bei 50-60°C, um ein Rohprodukt zu erhalten, Lösen des Rohprodukts in Methanol bei 30-65°C, Abkühlen auf Normaltemperatur und dann auf 0-5°C, Filtrieren, um einen kristallinen Feststoff zu erhalten, Waschen des kristallinen Feststoffs mit Methanol und anschließendes Trocknen des kristallinen Feststoffs bei 40-65°C.

3. Herstellungsverfahren nach Anspruch 1, worin das aprotische Lösungsmittel Toluol, N,N-Dimethylformamid oder Methylisobutylketon ist.

4. Herstellungsverfahren nach Anspruch 1, wobei das basische Gemisch 2-5 Stunden lang bei einer Temperatur von 80-120°C umgesetzt wird.

5. Herstellungsverfahren nach Anspruch 1, wobei in Schritt (a) das basische Gemisch für 3 Stunden bei einer Temperatur von 80°C umgesetzt wird; und in Schritt (b) die erste Destillation unter vermindertem Druck bei 80°C und 13332 Pa (100 Torr) erfolgt.

6. Herstellungsverfahren nach Anspruch 1, wobei in Schritt (a) das basische Gemisch für 2 Stunden bei einer Temperatur von 120°C umgesetzt wird; und in Schritt (b) die erste Destillation unter vermindertem Druck bei 80°C unter 13332 Pa (100 Torr) erfolgt.

7. Herstellungsverfahren nach Anspruch 1, wobei in Schritt (a),
das basische Gemisch bei 50-60°C unter 13332-15999 Pa (100-120 Torr) azeotrop vordestilliert wird, um Wasser zu entfernen; oder
die gemischten Komponenten des basischen Gemisches ohne das 1-Methylpyrrolidin vor der Zugabe von 1-Methylpyrrolidin bei 60°C unter 13332 Pa (100 Torr) azeotrop vordestilliert werden, um die basische Mischung zu bilden, wobei die basische Mischung 4 Stunden lang auf 80°C erhitzt wird.

## Revendications

1. Procédé de préparation d'azoxystrobine, le procédé étant **caractérisé en ce que** le procédé comprend les étapes (a) et (b):
(a) mélanger du (E)-2-[2-(6-chloropyrimidin-4-yloxy)phényl]-3-méthoxyacrylate de méthyle, du 2-cyanophénol, du carbonate de potassium, et 10-80% en moles de 1-méthylpyrrolidine comme catalyseur dans un solvant aprotique, pour former un mélange basique; et faire réagir le mélange basique pendant 2-5 heures à une température de 60-120°C; et
(b) soumettre le mélange basique de l'étape (a) à une première distillation sous une pression réduite de 10666-15999 Pa (80-120 Torr) à 70-80°C, pour obtenir l'azoxystrobine.

2. Procédé de préparation selon la revendication 1, comprenant en outre l'étape consistant à:
(c) ajouter le produit restant après la première distillation sous pression réduite au solvant aprotique et à l'eau, mélanger, et laisser reposer pour la séparation des couches, recueillir une couche organique supérieure, soumettre la couche organique à une seconde distillation sous une pression réduite de 2666-5333 Pa (20-40 Torr) à 50-60°C pour obtenir un produit brut, dissoudre le produit brut dans du méthanol à 30-65°C, refroidir à température normale et ensuite à 0-5°C, filtrer pour obtenir un solide cristallin, laver le solide cristallin avec du méthanol, et ensuite sécher le solide cristallin à 40-65°C.

3. Procédé de préparation selon la revendication 1, dans lequel le solvant aprotique est le toluène, le N,N-diméthylformamide, ou la méthylisobutylcétone.

4. Procédé de préparation selon la revendication 1, dans lequel le mélange basique est mis à réagir pendant 2 à 5 heures à une température de 80 à 120°C.

5. Procédé de préparation selon la revendication 1, dans lequel à l'étape (a), le mélange basique est mis à réagir pendant 3 heures à une température de 80°C; et à l'étape (b), la première distillation sous pression réduite a lieu à 80°C sous 13332 Pa (100 Torr).

6. Procédé de préparation selon la revendication 1, dans lequel à l'étape (a), le mélange basique est mis à réagir pendant 2 heures à une température de 120°C; et à l'étape (b), la première distillation sous pression réduite a lieu à 80°C sous 13332 Pa (100 Torr).

7. Procédé de préparation selon la revendication 1, dans lequel à l'étape (a),
le mélange basique est pré-distillé de manière azéotropique à 50-60°C sous 13332-15999 Pa (100-120 Torr) pour éliminer l'eau; ou
les composants mélangés du mélange basique sans la 1-méthylpyrrolidine sont prédistillés de manière azéotropique à 60°C sous 13332 Pa (100 Torr) avant d'ajouter la 1-méthylpyrrolidine pour former le mélange basique, dans lequel le mélange basique est chauffé à 80°C pendant 4 heures.
